Europäisches Patentamt

European Patent Office

Office européen des brevets

(11) Veröffentlichungsnummer: **0 480 280 B1**

## (12) EUROPÄISCHE PATENTSCHRIFT

(45) Veröffentlichungstag der Patentschrift: **22.02.95**

(51) Int. Cl.⁶: **C08F  220/18**

(21) Anmeldenummer: **91116654.4**

(22) Anmeldetag: **30.09.91**

(54) **Terpolymerisate, ihre Verwendung in Haarfestigungsmitteln mit erhöhter Festigungswirkung und diese enthaltende Haarfestigungsmittel.**

(30) Priorität: **08.10.90 DE 4031912**

(43) Veröffentlichungstag der Anmeldung:
**15.04.92 Patentblatt  92/16**

(45) Bekanntmachung des Hinweises auf die
Patenterteilung:
**22.02.95 Patentblatt  95/08**

(84) Benannte Vertragsstaaten:
**DE ES FR GB IT NL**

(56) Entgegenhaltungen:
**EP-A- 0 257 444**
**US-A- 3 405 084**

(73) Patentinhaber: **BASF Aktiengesellschaft**
**Carl-Bosch-Strasse 38**
**D-67063 Ludwigshafen (DE)**

(72) Erfinder: **Potthoff-Karl, Birgit, Dr.**
**Gruenerstrasse 7**
**W-6700 Ludwigshafen (DE)**
Erfinder: **Sperling-Vietmeier, Karin, Dr.**
**Im Kirchenstueck 12**
**W-6730 Neustadt (DE)**
Erfinder: **Frosch, Franz, Dr.**
**Auf dem Koeppel 112**
**W-6702 Bad Duerkheim (DE)**
Erfinder: **Sanner, Axel, Dr.**
**Lorscher Ring 2 c**
**W-6710 Frankenthal (DE)**

**Beschreibung**

Die vorliegende Erfindung betrifft ein Terpolymerisat, welches durch radikalische Polymerisation von
10 bis 24 Gew.-% N-Vinylpyrrolidon,
46 bis 74 Gew.-% tert.-Butylacrylat oder tert.-Butylmethacrylat und
16 bis 30 Gew.-% Acrylsäure oder Methacrylsäure
erhältlich ist, dessen Carboxylgruppen gegebenenfalls teilweise oder vollständig durch organische Amine, Ammoniak und/oder basische Alkalimetallverbindungen neutralisiert sind und das einen K-Wert von 25 bis 65 aufweist.

Weiterhin betrifft die Erfindung die Verwendung dieser Terpolymerisate in Haarfestigungsmitteln mit erhöhter Festigungswirkung sowie diese enthaltende Haarfestigungsmittel.

In der US-A 3 405 084 (1) werden neutralisierte Terpolymerisate für Haarsprayformulierungen beschrieben, die aus 25 bis 75 Gew.-% N-Vinylpyrrolidon, 20 bis 70 Gew.-% eines Alkylesters der Acryl- oder Methacrylsäure mit einem geradkettigen oder verzweigten Rest von 1 bis 10 C-Atomen im Esteralkohol und 3 bis 25 Gew.-% Acrylsäure oder Hethacrylsäure bestehen.

Die EP-A 257 444 (2) betrifft ein Terpolymerisat für Haarbehandlungsmittel, das durch radikalische Polymerisation aus 20 bis 50 Gew.-% N-Vinylpyrrolidon, 40 bis 70 Gew.-% tert-Butylacrylat oder tert.-Butylmethacrylat und 2 bis 15 Gew.-% Acrylsäure oder Methacrylsäure erhalten wird, dessen Carboxylgruppen gegebenenfalls zu 5 bis 100 % durch ein organisches Amin neutralisiert sind und das einen K-Wert von 10 bis 60 aufweist.

In jüngster Zeit gewinnen bei Filmbildnern in Haarbehandlungs- und Haarfestigungsmitteln neben den Kriterien der Curl Retention und der Verträglichkeit mit unpolaren Treibgasen wie Kohlenwasserstoffen und Dimethylether die Eigenschaften Wasserlöslichkeit und vor allem Auswaschbarkeit und Festigungswirkung immer mehr an Bedeutung, da der Trend von herkömmlichen wasserfreien Haarsprayzubereitungen zu wäßrigen Zubereitungen ohne oder mit wenig Treibgas übergeht. Eine gute Auswaschbarkeit wird beispielsweise deshalb angestrebt, weil schon geringe Restbestandteile von Polymeren im Haar zu Schwierigkeiten bei der Neugestaltung der Frisur führen. Von den Mitteln aus (2) ist bekannt, daß mit steigendem K-Wert, der als ein Maß für die Festigungswirkung angesehen werden kann, gleichzeitig die Auswaschbarkeit des filmbildenden Polymers aus dem Haar und die Wasserlöslichkeit des Polymers in den Zubereitungen deutlich schlechter werden.

Aufgabe der vorliegenden Erfindung war daher, ein Polymer für die Haarkosmetik bereitzustellen, welches bei größenordnungsmäßig vergleichbaren Werten für Curl Retention und Propan/Butan-Verträglichkeit und bei nur geringer oder nicht vorhandener Klebeneigung der auf dem Haar gebildeten Filme eine erhöhte Festigungswirkung bei gleichzeitiger guter Auswaschbarkeit und Wasserlöslichkeit aufweist.

Demgemäß wurde das eingangs definierte Terpolymerisat gefunden, das im Vergleich zu den Mitteln aus (2) höhere K-Werte aufweist, aber gleichzeitig gut auswaschbar und ausreichend wasserlöslich ist.

In einer bevorzugten Ausführungsform ist das erfindungsgemäße Terpolymerisat aus
10 bis 20 Gew.-% N-Vinylpyrrolidon,
55 bis 74 Gew.-% tert.-Butylacrylat oder tert.-Butylmethacrylat und
16 bis 25 Gew.-% Acrylsäure oder Methacrylsäure
aufgebaut.

Die erfindungsgemäßen Terpolymerisate werden vorteilhafterweise durch radikalisch ablaufende Polymerisationsreaktion unter den üblichen Bedingungen hergestellt. Man arbeitet hierbei nach den gängigen Techniken, zum Beispiel nach den Methoden der Suspensions-, Emulsion- oder Lösungspolymerisation.

Als besonders zweckmäßig hat sich die Lösungspolymerisation in einem organischen Lösungsmittel, in der Regel einem Alkohol, herausgestellt. Man arbeitet hier üblicherweise bei Temperaturen von 60 bis 160 °C, wobei die Umsetzung bei Normaldruck oder unter Eigendruck durchgeführt werden kann.

Um zu besonders niedrigen Restmonomergehalten zu gelangen, ist es von Vorteil, gemäß EP-B 000 161 (3) dem Reaktionsgemisch nach Beendigung der Hauptpolymerisation 0,05 bis 0,5 Gew.-%, bezogen auf die eingesetzten Monomeren, Di-tert.-butylperoxid, Di-tert.-amylperoxid, Dicumylperoxid, 2,5-Dimethyl-2,5-bis-(tert.-butylperoxy)-hexan, 2,2-Bis-(tert.-butylperoxy)-butan, 1,1-Bis(tert.-butylperoxy)-3,3,5-trimethylcyclohexan oder 4,4-Di-(tert.-butylperoxy)-butylvalerat zuzusetzen und die Nachpolymerisation bei höherer Temperatur als die Hauptpolymerisation im Bereich von 100 bis 200 °C durchzuführen.

Als Initiatoren für die radikalisch ablaufende Polymerisationsreaktion werden die üblichen Peroxo- oder Azoverbindungen, beispielsweise Diacylperoxide, wie z.B. Dibenzoylperoxid, Peroxyester, wie z.B. tert.-Butylperpivalat, tert.-Butylper-2-ethylhexanoat, Dialkylperoxide, wie z.B. Di-tert.-Butylperoxid, 2,5-Dimethyl-2,5-di(tert.-butyl-peroxy)hexan, Alkylhydroperoxide, wie z.B. tert.-Butylhydroperoxid, Azoverbindungen, wie z.B. Azo-bis-isobutyronitril, Azo-bis-isobutyromethylester, zweckmäßigerweise in Mengen von 0,1 bis 2

2

Gew.-%, bezogen auf das Gewicht der Monomeren, eingesetzt.

Man wählt die Mengen an Monomeren und Lösungsmittel zweckmäßigerweise so, daß man 30 bis 80 gew.-%ige Lösungen der Terpolymerisate erhält. Dabei kann vorliegendes organisches Lösungsmittel nach den üblichen Methoden, z.B. durch Destillation, entfernt und durch Wasser ersetzt werden, falls ein Haarfestigungsmittel auf wäßriger Basis gewünscht wird. Falls erforderlich, kann das Lösungsmittel auch auf übliche Weise, z.B. durch Sprühtrocknung, vollständig entfernt werden.

Die erfindungsgemäßen Terpolymerisate liegen vorzugsweise in durch organische Amine, Ammoniak und/oder durch basische Alkalimetallverbindungen teilweise oder vollständig neutralisierter Form vor, wobei die Carboxylgruppen zu 5 bis 100 % neutralisiert sind. Besonders bevorzugt ist eine Teilneutralisation der Carboxylgruppen von 50 bis 100 %, insbesondere von 70 bis 100 %.

Die Neutralisation der Carboxylgruppen durch organische Amine erfolgt bevorzugt mit einem Alkanolamin aus der Reihe der Mono-, Di- oder Trialkanolamine mit 2 bis 5 C-Atomen im Alkanolrest, wie Mono-, Di- oder Trietahanolamin, Mono-, Di- oder Tripropanolamin oder 2-Amino-2-methylpropanol, oder Alkandiolaminen mit 2 bis 4 C-Atomen im Alkandiolrest, wie 2-Amino-2-methyl-1,3-propandiol oder 2-Amino-2-ethyl-1,3-propandiol, oder durch Di (methoxyethyl)amin oder durch primäre, sekundäre und tertiäre Alkylamine mit insgesamt 5 bis 10 C-Atomen, wie N,N-Diethylpropylamin. Davon sind 2-Amino-2-methyl-propanol, Triisopropanolamin und 2-Amino-2-ethyl-1,3-propandiol bevorzugt.

Weiterhin kann die Neutralisation der Carboxylgruppen durch Ammoniak oder durch basische Alkalimetallverbindungen wie Alkalimetallhydroxide, z.B. Lithiumhydroxid oder insbesondere Natrium- und Kaliumhydroxid, Alkalimetallcarbonate, z.B. Natrium- und Kaliumcarbonat, oder Alkalimetallalkoholate, z.B. Natriummethylat und -ethylat, erfolgen. Es können auch Mischungen aus organischen Aminen, Ammoniak und basischen Alkalimetallverbindungen eingesetzt werden.

Die erfindungsgemäßen Terpolymeren weisen K-Werte von 25 bis 65, vorzugsweise 35 bis 55, gemessen in 1 gew.-%iger Lösung in Ethanol bei 25°C in der Säureform, auf. Der K-Wert ist ein Maß für das Molgewicht (Fikentscher, Cellulosechemie 13, (1932) Seite 58 bis 64 und 71 bis 74). Der K-Wert läßt sich prinzipiell durch die Wahl der Polymerisationsbedingungen einstellen.

Die erfindungsgemäßen Terpolymerisate eignen sich in hervorragender Weise als Filmbildner in Haarfestigungsmitteln für die Haarkosmetik. Derartige Haarfestigungsmittel sind deshalb weiterhin Gegenstand der vorliegenden Erfindung. Sie kommen beispielsweise als Haarfestigerlösungen, Haarschäume, Haargele oder als Frisurenfestiger wie Sprayzubereitungen zur Anwendung.

Die erfindungsgemäßen Haarfestigungsmittel enthalten vorzugsweise

- 1 bis 15 Gew.-%, insbesondere 2 bis 10 Gew.-%, eines erfindungsgemäßen Terpolymerisates,
- 10 bis 99 Gew.-% eines Lösungsmittels aus der Gruppe Wasser, Aceton, Ethanol, n-Propanol, n-Butanol, Isopropanol und 1-Methoxypropanol oder deren Gemische, wobei die Verwendung von Wasser, Ethanol und Isopropanol besonders günstig ist, sowie
- 0 bis 90 Gew.-% eines Treibmittels (Treibgas) aus der Gruppe Propan, n-Butan, Isobutan, 2,2-Dimethylpropan, Isopentan und Dimethylether und deren Gemische, wobei die Verwendung von Propan und n-Butan sowie einer Mischung hieraus, z.B. im Gewichtsverhältnis von 40 : 60 oder 25 : 75, besonders günstig ist.

Daneben kommen weitere übliche Zusätze, wie Parfüm, Konservierungsstoffe u. a., in den hierfür üblichen Mengen in Betracht.

Eine zweckmäßige Zubereitung für Haarfestigerlösungen enthält beispielsweise:

- 1 bis 15 Gew-% erfindungsgemäßes Terpolymerisat, dessen Carboxylgruppen.zu 5 bis 100 %, vorzugsweise 50 bis 100 %, neutralisiert sind,
- 0 bis 99 Gew.-% eines Lösungsmittels aus der Gruppe Aceton, Ethanol, Propanol, n-Butanol, Isopropanol und 1-Methoxypropanol-2 oder deren Gemische und
- 0 bis 99 Gew.-% Wasser.

Eine bevorzugte Zusammensetzung für Haarfestigerlösungen ist überwiegend wäßrig und enthält 2 bis 10 Gew.-% des Terpolymerisats und 60 bis 98 Gew.-% Wasser und gegebenenfalls als Rest zu 100 Gew.-% eines der obengenannten Lösungsmittel oder deren Gemische.

Eine zweckmäßige und vorteilhafte Zusammensetzung für Haarschäume ergibt sich nach folgender Vorschrift:

- 1 bis 15 Gew.-%, vorzugsweise 5 bis 10 Gew.-%, erfindungsgemäßes Terpolymerisat, dessen Carboxylgruppen zu 5 bis 100 %, vorzugsweise 50 bis 100 Gew.-%, neutralisiert sind,
- 5 bis 90 Gew.-%, vorzugsweise 60 bis 80 Gew.-% Wasser,
- 0 bis 15 Gew.-%, vorzugsweise 5 bis 10 Gew.-% eines Lösungsmittels aus der Gruppe Aceton, Ethanol, n-Propanol, n-Butanol, Isopropanol und 1-Methoxypropanol-2 oder deren Gemische, und

- 50 bis 20 Gew.-% eines Treibmittels aus der Gruppe Propan, n-Butan, Isobutan, 2,2-Dimethylpropan, Isopentan und Dimethylether und deren Gemische, wovon Mischungen aus Propan/Butan bevorzugt sind.

Diesen Zusammensetzungen werden, bezogen auf das Gesamtgewicht 0,5 bis 1 Gew.-% dem Fachmann an sich bekannte Hilfsmittel zur Schaumbildung und Schaumstabilisierung zugesetzt.

Eine zweckmäßige Haarsprayzusammensetzung enthält beispielsweise:

- 1 bis 15 Gew.-% erfindungsgemäßes Terpolymerisat, dessen Carboxylgruppen zu 5 bis 100 %, vorzugsweise 50 bis 100 %, neutralisiert sind,
- 10 bis 95 Gew.-% eines Lösungsmittels aus der Gruppe Aceton, Ethanol, n-Propanol, n-Butanol, Isopropanol und 1-Methoxypropanol-2 oder deren Gemische und
- 5 bis 90 Gew.-% eines Treibmittels aus der Gruppe Propan, n-Butan, Isobutan, 2,2-Dimethylpropan, Isopentan und Dimethylether und deren Gemische.

Die üblichen Bestandteile und Zusammensetzungen anderer Haarfestigungsmittel sind dem Fachmann bekannt und brauchen deshalb hier nicht näher erläutert zu werden.

Die erfindungsgemäßen Terpolymerisate zeichnen sich durch eine hervorragende Festigungswirkung als Filmbildner in Haarfestigungsmitteln für die Haarkosmetik aus, wobei sie gleichzeitig gut aus dem Haar wieder auswaschbar sind und eine ausreichende Wasserlöslichkeit in den haarkosmetischen Zubereitungen aufweisen. Die Zubereitungen sind in der Regel klar bis opak. Weiterhin kleben die erfindungsgemäßen Terpolymerisate auf dem Haar nicht und zeigen für Curl-Retention und Propan/Butan-Verträglichkeit für die Praxis ausreichende Werte in der Größenordnung wie vergleichbare Mittel des Standes der Technik.

Beispiel 1

Herstellung eines Terpolymerisats aus 20 Gew.-% N-Vinylpyrrolidon, 60 Gew.-% tert.-Butylacrylat und 20 Gew.-% Methacrylsäure

20 g N-Vinylpyrrolidon, 60 g tert.-Butylacrylat und 20 g Methacrylsäure werden in 24 g Ethanol gelöst. In einem Rührkolben mit Rückflußkühler und 2 Tropftrichtern wurden 10 % dieser Monomerenlösung, 10 % einer vorbereiteten Starterlösung aus 0,8 g tert.-Butylperpivalat in 27 g Ethanol und 50 g Ethanol vorgelegt und die Mischung wurde auf ca. 75°C aufgeheizt.

Nach dem Anpolymerisieren, erkennbar an einer Viskositätserhöhung, wurde die restliche Monomerlösung innerhalb von 3 Stunden und gleichzeitig die restliche Starterlösung innerhalb von etwa 6 Stunden zugegeben, wobei die Innentemperatur auf ca. 78 bis 80°C gehalten wurde.

Nach dem Ende der Zugabe wurde noch ca. 1 Stunde bei diesen Temperaturen nachpolymerisiert.

Beispiele 2 bis 6 und Vergleichsbeispiele A bis C

Analog Beispiel 1 wurden die erfindungsgemäßen Terpolymerisate der in der Tabelle angebenen Zusammensetzung hergestellt. Die Herstellung der Vergleichssubstanzen A bis C erfolgte gemäß Literaturstelle (2).

Anwendungstechnische Prüfung

Als Maß für die Festigungswirkung (engl. stiffness) wurde der Biegetest gemäß der Literaturstelle Parfums, cosmetiques, arômes n° 89, Octobre-Novembre 1989, 71, welcher auch auf dem BASF-Symposium "Cosmeticon" am 10.-11. Mai 1990 in Heidelberg vorgestellt wurde, durchgeführt. Dieser Test gibt an, welche Kraft nötig ist, um eine mit der filmbildenden Polymerlösung behandelte Haarsträhne bis zum Bruch der Filmhaut durchzubiegen. Je größer die Kraft ist, desto höher ist die Festungswirkung.

Für die Beurteilung der Auswaschbarkeit von Polymeren aus dem Haar wurde folgende Vorschrift zugrundegelegt:

Die Haarsträhnen werden mit einer 3 gew.-%igen ethanolischen Lösung des Polymers benetzt und anschließend an der Luft getrocknet. Danach werden die Haarsträhnen in einer 10 gew.-%igen wäßrigen Natriumlaurylethersulfat-Lösung bei 35 bis 40°C für die Dauer von 30 sec auf- und abbewegt. Dabei werden die Haare nur kurz gedrückt, um den Polymerfilm zu brechen, jedoch nicht intensiv gerieben. Die Haarsträhnen werden mit lauwarmen Wasser abgespült und der Vorgang des Waschens mit der oberflächenaktiven Lösung wird noch einmal wiederholt. Nach dem letzten Abspülen werden die Haare zwischen Filterpapier abgedrückt und abschließend an der Luft getrocknet. - Die Beurteilung erfolgt visuell; dabei werden die Haare gezwirbelt, um eventuell noch anhaftendes Polymer sichtbar zu machen. Die Beurteilungsskala reicht von "gut" über "noch gut" bis "schlecht".

Die folgende Tabelle zeigt neben der Zusammensetzung der Terpolymerisate und deren K-Werten die Ergebnisse des Biegetests und der Auswaschbarkeits-Prüfung sowie die Wasserlöslichkeit. Die drei Anwendungstests wurden jeweils mit einem zu 100 % neutralisierten Polymer durchgeführt.

| Versuch Nr. | Zusammensetzung [Gew.-%] VP | tBA | MAS | K-Wert | Biegetest [pond] | Auswasch-barkeit | Wasserlöslich-keit |
|---|---|---|---|---|---|---|---|
| 1 | 20 | 60 | 20 | 45 | 117 | gut | klar löslich |
| 2 | 10 | 70 | 20 | 53 | 117 | gut | blaustichig löslich |
| 3 | 15 | 69 | 16 | 40 | 111 | gut | opak |
| 4 | 12 | 70 | 18 | 42 | 108 | gut | schwach opak |
| 5 | 19 | 65 | 16 | 38 | 107 | gut | trübe |
| 6 | 20 | 55 | 25 | 40 | 104 | gut | klar löslich |
| Zum Vergleich: | | | | | | | |
| A | 20 | 70 | 10 | 24 | 48 | gut | klar löslich |
| B | 20 | 70 | 10 | 37 | 92 | schlecht | trübe, Bodensatz |
| C | 20 | 70 | 10 | 44 | 106 | schlecht | trübe, Bodensatz |

VP: N-Vinylpyrroliodon, tBA: tert.-Butylacrylat, MAS: Methacrylsäure

Die Substanzen der Vergleichsbeispiele A bis C sind gemäß Literaturstelle (2).

Das Terpolymer des Bsp. 1 hat eine Curl Retention von 88 % und eine Propan/Butan-Verträglichkeit von 54 % (Zum Vergleich Bsp. A: 90 % Curl Retention und 72 % Propan/Butan-Verträglichkeit).

**Patentansprüche**
**Patentansprüche für folgende Verstragsstaaten : DE, FR, GB, IT, NL**

1. Terpolymerisat, erhältlich durch radikalische Polymerisation von
10 bis 24 Gew.-% N-Vinylpyrrolidon,
46 bis 74 Gew.-% tert.-Butylacrylat oder tert.-Butylmethacrylat und
16 bis 30 Gew.-% Acrylsäure oder Methacrylsäure,
dessen Carboxylgruppen gegebenenfalls teilweise oder vollständig durch organische Amine, Ammoniak und/oder basische Alkalimetallverbindungen neutralisiert sind und das einen K-Wert von 25 bis 65 aufweist.

2. Terpolymerisat nach Anspruch 1, erhältlich durch radikalische Polymerisation von
10 bis 20 Gew.-% N-Vinylpyrrolidon,
55 bis 74 Gew.-% tert.-Butylacrylat oder tert.-Butylmethacrylat und
16 bis 25 Gew.-%, Acrylsäure oder Methacrylsäure,
und dessen Carboxylgruppen zu 5 bis 100 % durch organische Amine, Ammoniak und/oder durch basische Alkalimetallverbindungen neutralisiert sind und das einen K-Wert von 35 bis 55 aufweist.

3. Haarfestigungsmittel, enthaltend neben den hierfür üblichen Bestandteilen als Filmbildner ein Terpolymerisat gemäß Anspruch 1 oder 2.

4. Haarfestigungsmittel, enthaltend 1 bis 15 Gew.-% eines Terpolymerisats gemäß Anspruch 1 oder 2, 10 bis 99 Gew.-% eines Lösungsmittels aus der Gruppe Wasser, Aceton, Ethanol, n-Propanol, n-Butanol, Isopropanol und 1-Methoxypropanol oder deren Gemische und 0 bis 90 Gew.-% eines Treibmittels aus der Gruppe Propan, n-Butan, Isobutan, 2,2-Dimethylpropan, Isopentan und Dimethylether und deren Gemische.

5. Verwendung eines Terpolymerisats gemäß Anspruch 1 oder 2 als Filmbildner in Haarfestigungsmitteln.

**Patentansprüche für folgenden Verstragsstaat : ES**

1. Verfahren zur Herstellung eines Terpolymerisates, dadurch gekennzeichnet, daß man
10 bis 24 Gew.-% N-Vinylpyrrolidon,
46 bis 74 Gew.-% tert.-Butylacrylat oder tert.-Butylmethacrylat und
16 bis 30 Gew.-% Acrylsäure oder Methacrylsäure
radikalisch polymerisiert und die Carboxylgruppen gegebenenfalls teilweise oder vollständig durch organische Amine, Ammoniak und/oder basische Alkalimetallverbindungen neutralisiert, wobei das Terpolymerisat einen K-Wert von 25 bis 65 aufweist.

2. Verfahren nach Anspruch 1, dadurch gekennzeichnet, daß man
10 bis 20 Gew.-% N-Vinylpyrrolidon,
55 bis 74 Gew.-% tert.-Butylacrylat oder tert.-Butylmethacrylat und
16 bis 25 Gew.-%, Acrylsäure oder Methacrylsäure
radikalisch polymerisiert und die Carboxylgruppen zu 5 bis 100 % durch organische Amine, Ammoniak und/oder durch basische Alkalimetallverbindungen neutralisiert, wobei das Terpolymerisat einen K-Wert von 35 bis 55 aufweist.

3. Verfahren zur Herstellung eines Haarfestigungsmittels, dadurch gekennzeichnet, daß man hierfür übliche Bestandteile mit einem Terpolymerisat gemäß Anspruch 1 oder 2 als Filmbildner mischt.

4. Verfahren nach Anspruch 3, dadurch gekennzeichnet, daß man 1 bis 15 Gew.-% eines Terpolymerisats gemäß Anspruch 1 oder 2, 10 bis 99 Gew.-% eines Lösungsmittels aus der Gruppe Wasser, Aceton, Ethanol, n-Propanol, n-Butanol, Isopropanol und 1-Methoxypropanol oder deren Gemische und 0 bis 90 Gew.-% eines Treibmittels aus der Gruppe Propan, n-Butan, Isobutan, 2,2-Dimethylpropan, Isopentan und Dimethylether und deren Gemische miteinander mischt.

## Claims
## Claims for the following Contracting States : DE, FR, GB, IT, NL

1. A terpolymer obtainable by free radical polymerization of 10-24 % by weight of N-vinylpyrrolidone, 46-74 % by weight of tert-butyl acrylate or methacrylate and 16-30 % by weight of acrylic or methacrylic acid,
whose carboxyl groups may be partially or completely neutralized with an organic amine, ammonia and/or a basic alkali metal compound and which has a K value of from 25 to 65.

2. A terpolymer as claimed in claim 1, obtainable by free radical polymerization of 10-20 % by weight of N-vinylpyrrolidone, 55-74 % by weight of tert-butyl acrylate or methacrylate and 16-25 % by weight of acrylic or methacrylic acid,
whose carboxyl groups have been neutralized with an organic amine, ammonia and/or a basic alkali metal compound to an extent of from 5 to 100 % and which has a K value of from 35 to 55.

3. A hair setting composition, containing as film former a terpolymer as claimed in claim 1 or 2, as well as customary ingredients for this purpose.

4. A hair setting composition, containing from 1 to 15 % by weight of a terpolymer as claimed in claim 1 or 2, from 10 to 99 % by weight of a solvent selected from the group consisting of water, acetone, ethanol, n-propanol, n-butanol, isopropanol, 1-methoxypropanol and mixtures thereof and from 0 to 90 % by weight of a propellant selected from the group consisting of propane, n-butane, isobutane, 2,2-dimethylpropane, isopentane, dimethyl ether and mixtures thereof.

5. The use as a film former in hair setting compositions of a terpolymer as claimed in claim 1 or 2.

## Claims for the following Contracting State : ES

1. A process for preparing a terpolymer, which comprises free radical polymerization of 10-24 % by weight of N-vinylpyrrolidone, 46-74 % by weight of tert-butyl acrylate or methacrylate and 16-30 % by weight of acrylic or methacrylic acid, and partially or completely neutralizing the carboxyl groups with an organic amine, ammonia and/or a basic alkali metal compound, the terpolymer having a K value of from 25 to 65.

2. A process as claimed in claim 1, wherein 10-20 % by weight of N-vinylpyrrolidone, 55-74 % by weight of tert-butyl acrylate or methacrylate and 16-25 % by weight of acrylic or methacrylic acid are subjected to free radical polymerization and the carboxyl groups are neutralized with an organic amine, ammonia and/or a basic alkali metal compound to an extent of from 5 to 100 %, the terpolymer having a K value of from 35 to 55.

3. A process for producing a hair setting composition, which comprises mixing customary ingredients for this purpose with a terpolymer as claimed in claim 1 or 2 as a film former.

4. A process as claimed in claim 3, wherein from 1 to 15 % by weight of a terpolymer as claimed in claim 1 or 2, from 10 to 99 % by weight of a solvent selected from the group consisting of water, acetone, ethanol, n-propanol, n-butanol, isopropanol, 1-methoxypropanol and mixtures thereof and from 0 to 90 % by weight of a propellant selected from the group consisting of propane, n-butane, isobutane, 2,2-dimethylpropane, isopentane, dimethyl ether and mixtures thereof are mixed with one another.

## Revendications
## Revendications pour les Etats contractants suivants : DE, FR, GB, IT, NL

1. Terpolymère, qui est obtenu par polymérisation radicalaire de
10 à 24% en poids de N-vinylpyrrolidone,
46 à 74% en poids d'acrylate de tert.-butyle ou de méthacrylate de tert.-butyle et
16 à 30% en poids d'acide acrylique ou d'acide méthacrylique,
dont les groupements carboxyle sont éventuellement neutralisés partiellement ou complètement par des amines organiques, de l'ammoniac et/ou des composés basiques de métaux alcalins et qui présente une valeur K de 25 à 65.

2. Terpolymère selon la revendication 1, qui est obtenu par polymérisation radicalaire de
10 à 20% en poids de N-vinylpyrrolidone,
55 à 74% en poids d'acrylate de tert.-butyle ou de méthacrylate de tert.-butyle et
16 à 25% en poids d'acide acrylique ou d'acide méthacrylique,
dont les groupements carboxyle sont neutralisés à raison de 5 à 100% par des amines organiques, de l'ammoniac et/ou par des composés basiques de métaux alcalins et qui présente une valeur K de 35 à 55.

3. Fixateur pour cheveux, contenant, en dehors des constituants usuels pour un tel produit, un terpolymère selon la revendication 1 ou 2 en tant que filmogène.

4. Fixateur pour cheveux, contenant 1 à 15% en poids d'un terpolymère selon la revendication 1 ou 2, 10 à 99% en poids d'un solvant du groupe constitué par l'eau, l'acétone, l'éthanol, le n-propanol, le n-butanol, l'isopropanol et le 1-méthoxypropanol ou des mélanges de ceux-ci, et 0 à 90% en poids d'un agent propulseur du groupe constitué par le propane, le n-butane, l'isobutane, le 2,2-diméthylpropane, l'isopentane et l'oxyde de diméthyle ou des mélanges de ceux-ci.

5. Utilisation d'un terpolymère selon la revendication 1 ou 2 comme filmogène dans des fixateurs pour cheveux.

**Revendications pour l'Etat contractant suivant : ES**

1. Procédé de préparation d'un terpolymère, caractérisé en ce qu'on soumet à une polymérisation radicalaire
10 à 24% en poids de N-vinylpyrrolidone,
46 à 74% en poids d'acrylate de tert.-butyle ou de méthacrylate de tert.-butyle et
16 à 30% en poids d'acide acrylique ou d'acide méthacrylique,
et on neutralise éventuellement les groupements carboxyle, partiellement au complètement, par des amines organiques, de l'ammoniac et/ou des composés basiques de métaux alcalins, le terpolymère présentant une valeur K de 25 à 65.

2. Procédé selon la revendication 1, caractérisé en ce qu'on soumet à une polymérisation radicalaire
10 à 20% en poids de N-vinylpyrrolidone,
55 à 74% en poids d'acrylate de tert.-butyle ou de méthacrylate de tert.-butyle et
16 à 25% en poids d'acide acrylique ou d'acide méthacrylique,
et on neutralise les groupements carboxyle, à raison de 5 à 100%, par des amines organiques, de l'ammoniac et/ou des composés basiques de métaux alcalins, le terpolymère présentant une valeur K de 35 à 55.

3. Procédé de préparation d'un fixateur pour cheveux, caractérisé en ce qu'on mélange des constituants usuels pour un tel produit avec un terpolymère selon la revendication 1 ou 2 en tant que filmogène.

4. Procédé selon la revendication 3, caractérisé en ce qu'on mélange 1 à 15% en poids d'un terpolymère selon la revendication 1 ou 2, 10 à 99% en poids d'un solvant du groupe constitué par l'eau, l'acétone, l'éthanol, le n-propanol, le n-butanol, l'isopropanol et le 1-méthoxypropanol ou des mélanges de ceux-ci et 0 à 90% en poids d'un agent propulseur du groupe constitué par le propane, le n-butane, l'isobutane, le 2,2-diméthylpropane, l'isopentane et l'oxyde de diméthyle ou des mélanges de ceux-ci.